# EUROPEAN PATENT APPLICATION

(11) **EP 4 176 892 A1**
(43) Date of publication of application: **10.05.2023**
(21) Application number: 21833782.2
(22) Date of filing: 02.07.2021
(51) Int. Cl.: A61K 36/888, A61K 36/8884, A61K 36/8888, A61P 25/00, A61P 25/02, A61P 25/28

(54) **USE OF ARACEAE PLANT EXTRACT IN PREPARATION OF MEDICINE FOR TREATING NERVE INJURY**

(30) Priority: 02.07.2020 US 202063047586 P
(71) Applicant: National Tsing Hua University, Hsinchu City 30013 (TW)
(72) Inventor: WANG, Chen, Hsinchu, Taiwan 30013 (CN); LIAO, Wen-Ling, Hsinchu, Taiwan 30013 (CN); WANG, Yi, Hsinchu, Taiwan 30013 (CN); LI, Chia-Wei, Hsinchu, Taiwan 30013 (CN); YEN, Chia-Hung, Hsinchu, Taiwan 30013 (CN); CHANG, Chu-Yuan, Hsinchu, Taiwan 30013 (CN); WANG, Sheng-Yang, Hsinchu, Taiwan 30013 (CN); LIANG, Min-Zong, Hsinchu, Taiwan 30013 (CN); HUANG, Pei-Yuan, Hsinchu, Taiwan 30013 (CN); CHEN, Linyi, Hsinchu, Taiwan 30013 (CN)
(74) Representative: karo IP
(86) International application number: PCT/CN2021/104380
(87) International publication number: WO 2022/002271

(57) **Abstract**

Araceae plant extract is firstly used to promote neuronal repair, and Monstera epipremnoides extract is used for promoting neuronal regeneration after brain trauma. It is confirmed that the Monstera epipremnoides extract can promote regeneration of cortical neurons and hippocampal neurons after brain trauma; and the Monstera epipremnoides extract can promote the recovery of motor neuron function in mice after brain trauma; and it is further confirmed that the Monstera epipremnoides extract has no direct effect on glial cells.

## Description

### FIELD OF THE INVENTION

The invention relates to a use of an Araceae extract for promoting regeneration of injured neurons.

### BACKGROUND OF THE INVENTION

About 70 million people suffer from neuronal injury, such as traumatic brain injury (TBI), around the world annually. The common therapeutic methods include physiotherapy, hyperbaric oxygenation, transcranial magnetic stimulation and transcranial direct current stimulation. This kind of noninvasive therapy can improve the depression and cognitive function after TBI, but currently there is no effective drug for promoting neuronal regeneration after brain injury.

The TBI is induced by an external force impacting the brain, and there are about 70 million diagnosed cases all over the world annually. TBI can damage brain neurons and lead to motor function deficits and cognitive dysfunction of patients. The injured central nervous system is hard to regenerate, and there is not yet a medically effective therapy on promoting neuronal regeneration for TBI patients. Patients with a severe brain trauma are likely to develop neuronal degeneration in the future. Therefore, treating TBI patients early is the therapeutic solution.

Moreover, in terms of selection of therapeutic drugs, as the brain has a blood-brain barrier, not all drugs can penetrate the blood-brain barrier freely to achieve an effective dose. Hence, effective drug dosing becomes a serious problem in treating brain lesions.

Additionally, the Araceae are mostly toxic, such as Dieffenbachia, Arisaema, Alocasia or Monstera. Thus, they had not been considered as drugs before.

In view of this, it is urgent to develop a drug for treating neuronal injury, and a drug that can penetrate the blood-brain barrier is needed urgently.

### SUMMARY OF THE INVENTION

It is to be understood that both the afore-mentioned general description and the following detailed description are exemplary and explanatory only and are not restrictive of the invention.

The human nervous system is divided into central nervous system and peripheral nervous system. The trigeminal nerve is the cranial nerve of peripheral nervous system and connected to the pons (central nervous system). Therefore, when the extract of the present invention passes through the olfactory epithelial cells through the nasal mucous membrane, enters the passages around olfactory and trigeminal nerves and enters the brain, the systemic neuron repair can be achieved through peripheral neurons.

In view of this, brain neurons that are difficult to be repaired are taken as experimental targets in the present invention to achieve successful repair of both the central and peripheral nervous systems.

The central nervous system is composed of brain and spinal cord. The central nervous system stated in the present invention includes, but not limited to, rhinencephalon, amygdaloid, hippocampus, neocortex, lateral ventricle, epithalamus, thalamus, hypothalamus, subthalamus, hypophysis, conarium, third ventricle, tectum of midbrain, cerebral peduncle, pretectum, cerebral aqueduct, pons, cerebellomedullary cistern and spinal cord.

The peripheral nervous system is composed of somatic neurons, and autonomic neurons. The peripheral nervous system stated in the present invention includes, but not limited to sensory neurons, motor neurons, cranial nerves, and spinal neurons, sympathetic neurons, parasympathetic neurons, and enteric nervous system.

The present invention uses Araceae extracts for treating neuronal injury. To let the persons with general knowledge of the domain understand the content of patent application, the present invention uses the extract of Monstera epipremnoides of Araceae extracts as embodiments.

In some embodiments, the Araceae extract is Monstera epipremnoides extract.

In some embodiments, the Monstera epipremnoides extract can promote neuronal regeneration, increase in number of neurons or neuronal repair in a subject.

In some embodiments, the Monstera epipremnoides extract can penetrate the blood-brain barrier and enter the brain to promote the neuron repair.

In some embodiments, the Monstera epipremnoides extract can promote the regeneration of cortical neurons and hippocampal neurons.

In some perfect embodiments, the concentration of Monstera epipremnoides extract is 0.035 to 35 mg/kg. In a perfect embodiment the Monstera epipremnoides extract is 3.5 mg/kg.

In the present invention, the Araceae extract can significantly promote neuronal repair, and the extract can penetrate the blood-brain barrier and enter the brain to promote the brain neurons repair, regeneration, or increase in number of neurons; as well as promote the regeneration of cortical neurons and hippocampal neurons.

Further, the present invention also provides an Araceae extract. In an embodiment, the Araceae extract comprises the compound A of chromatogram with at least one peak at 13.70 to about 14.10 minutes retention time determined by HPLC. In an embodiment, the Araceae extract comprises a compound B of chromatogram with at least one peak at 4.4 to about 4.6 minutes retention time determined by HPLC. In an embodiment, the Araceae extract comprises a compound C of chromatogram with at least one peak at 4.7 to about 5 minutes retention time determined by HPLC. In an embodiment, the Araceae extract comprises a compound D of chromatogram with at least one peak at 10 to about 11 minutes retention time determined by HPLC.

In an embodiment, the Araceae extract in the present invention is prepared through the following steps:
(A) the Araceae is extracted with an organic solvent, and filtered to obtain a filtrate A, after that the filtrate A is concentrated by rotary evaporation to obtain an extract A; and
(B) the extract A is analyzed at 210-400 nm by HPLC.

In an embodiment, the organic solvent of step (A) is ethyl acetate or methanol. In a perfect embodiment, the extract is water extract or an ethyl acetate extract.

It is to be understood that both the foregoing general description and the following detailed description are exemplary and explanatory only and are not restrictive of the invention.

### BRIEF DESCRIPTION OF THE DRAWINGS

**Fig. 1** shows that 18 plants with high effectiveness on the regeneration of neurites screened in the present invention.
**Fig. 2** shows the result of secondary screening of the top 18 plants that can promote the regeneration of neurites in the present invention.
**Fig. 3** shows the result of the effect of Monstera epipremnoides extract on neuronal repair.
**Fig. 4** shows the controlled cortical impact (CCI) model used in example 4.
**Fig. 5** shows the rearing test for the mouse in the cylinder in the dark in example 4.
**Fig. 6** shows the result of cylinder test in example 4.
**Fig. 7** shows the regeneration process of brain neurons promoted by Monstera epipremnoides water extract (MeE_{W}) in 3D brain slice tissue culture.
**Fig. 8** shows the result of using Monstera epipremnoides water extract (MeE_{W} to promote the regeneration of brain neurons in the present invention.
**Fig. 9** proves that the Monstera epipremnoides crude extract (MeE) and Monstera epipremnoides water extract (MeE_{W}) of the present invention will not influence glial cells.
**Fig. 10** shows the results of calculating the neuron cell counts in the injured brain area.
**Fig. 11** shows HPLC-ELSD result of Monstera epipremnoides extract of the present invention.

### DETAILED DESCRIPTION OF THE INVENTION

Additional specific embodiments of the present invention include, but are not limited to the following:

### EXAMPLE 1

### Screening effective plant extracts

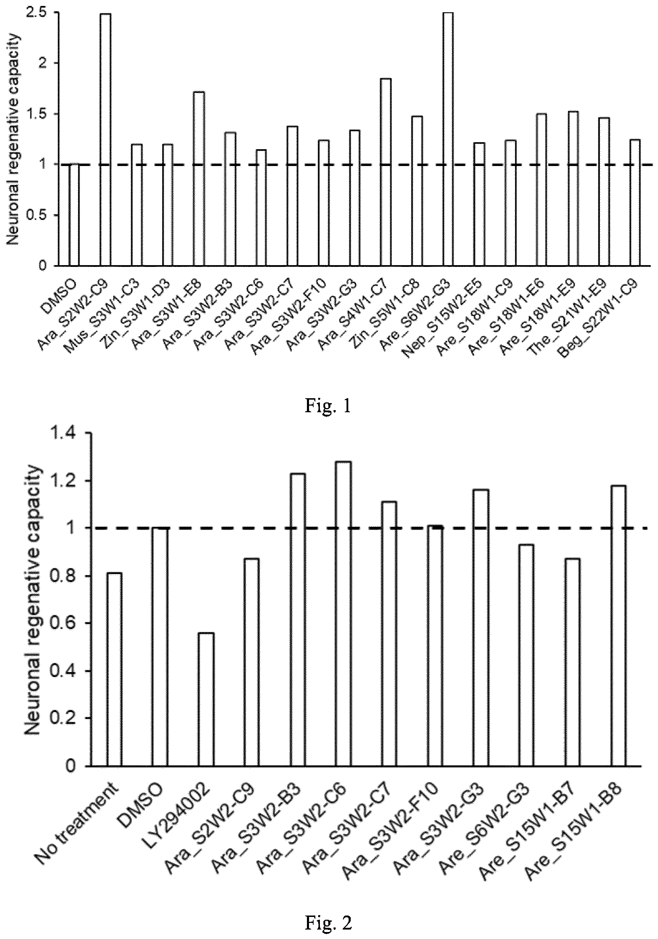

This embodiment was derived from the stems and leaves of plants provided by Dr. Cecilia Koo Botanic Conservation Center, which were extracted by Kaohsiung Medical University (extraction method was the same as example 2). The obtained 2,112 extracts were screened for the effectiveness on the regeneration of neurites. The regeneration of neurites was quantified by using the gap closure rate. The 2,112 plant extracts included 80 families, 845 kinds of plants, and 20 most effective extracts were obtained, most of which came from Araceae plants, as shown in Table 1 and Figure 1.

**Table 1. 18 plants with high effectiveness on the regeneration of neurites**

| **Screening code** | **KBCC code** | **Family** | **Genus** |
|---|---|---|---|
| Ara_S2W2-C9 | K007542 | Araceae | *Rhaphidophora sp.* |
| Mus_S3W1-C3 | K065229-MeOH | Musaceae | *Musa coccinea* |
| Zin_S3W1-D3 | K040019-MeOH | Zingiberaceae | *Hedychium sp.* |
| Ara_S3W1-E8 | K001349-Hex | Araceae | *Anthurium sp. nov. (Calomystrium)* |
| Ara_S3W2-B3 | K004898-EA | Araceae | *Rhaphidophora megaphylla* |
| Ara_S3W2-C6 | K001346 | Araceae | *Monstera epipremnoides* |
| Ara_S3W2-C7 | K001346-EA | Araceae | *Monstera epipremnoides* |
| Ara_S3W2-F10 | K004952 | Araceae | *Philodendron linnaei* |
| Ara_S3W2-G3 | K007290 | Araceae | *Dieffenbachia standleyi* |
| Ara_S4W1-C7 | K001339-Cr | Araceae | *Epipremnum sp.* |
| Zin_S5W1-C8 | K041198-H2O | Zingiberaceae | *Alpinia oui* |
| Are_S6W2-G3 | K008731-BuOH | Arecaceae | *Attalea* |
| Nep_S15W2-E5 | K046498-H2O | Nepenthaceae | *Nepenthes viking x macfarlanei* |
| Are_S18W1-C9 | K008757-Hex | Arecaceae | *Elaeis guineensis* |
| Are_S18Wl-E6 | K008840-H2O | Arecaceae | *Calamus sp.* |
| Are_S18Wl-E9 | K008865-H2O | Arecaceae | *Corypha umbraculifera* |
| The_S21W1-E9 | K002487-Hex | Theaceae | *Camellia fluviatilis* |
| Beg_S22W1-C9 | K031293-Hex | Begoniaceae | *Begonia silletensis* |

The regeneration of neurites is evaluated by gap closure rate for the first 18 candidate plant extracts, 15 better extracts are obtained by secondary screening, and Monstera epipremnoides plant of Araceae has the best regenerative effect, as shown in Figure 2 and Table 2.

**Table 2. 9 plants with higher effectiveness on the regeneration of neurites**

| **Screening code** | **KBCC code** | **Family** | **Genus** |
|---|---|---|---|
| Ara_S2W2-C9 | K007542 | Araceae | *Rhaphidophora sp.* |
| Ara_S3W2-B3 | K004898 EA | Araceae | *Rhaphidophora megaphylla* |
| Ara_S3W2-C6 | K001346 | Araceae | *Monstera epipremnoides* |
| Ara_S3W2-C7 | K001346 EA | Araceae | *Monstera epipremnoides* |
| Ara_S3W2-F10 | K004952 | Araceae | *Philodendron linnaei* |
| Ara_S3W2-G3 | K007290 | Araceae | *Dieffenbachia standleyi* |
| Are_S6W2-G3 | K008731 BuOH | Arecaceae | *Attalea* |
| Are_S15W1-B7 | K008802(H2O) | Arecaceae | *phalerata* |
| Are_S15W1-B8 | K008820(H2O) | Arecaceae | *paludosa* |

In order to find out the effect of Araceae extract of the present invention on promoting neuronal repair, the present invention uses Monstera epipremnoides extract as a representative for subsequent biologic tests.

### EXAMPLE 2

### Monstera epipremnoides extract preparation method

First, 900g branches and leaves of Monstera epipremnoides plant were cut into small pieces and soaked in ≥99.6% methanol for 3 to 5 days, filtered (by the known physical method, e.g. hole size, weight), and concentrated by a rotary evaporator at 45°C to obtain a Monstera epipremnoides crude extract (MeE). Afterwards, it was mixed with water and ≥99.5% ethyl acetate for partition by using polarity differences, and divided into water layer (MeE_{W}) and ethyl acetate layer (MeE_{EA}). The rotary evaporator concentration procedure was performed. The water layer and ethyl acetate layer were redissolved with DMSO to obtain a water extract (MeE_{W}) and an ethyl acetate extract (MeE_{EA}). The extracts were dissolved in ammonium formate and analyzed by HPLC as shown in Table 3.

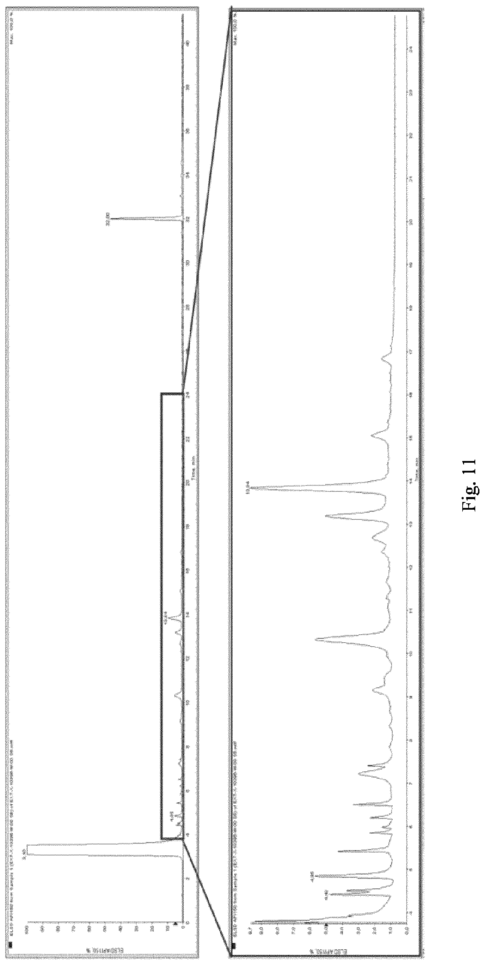

The two-step purified crude sample was analyzed by preparative separation. The MeE_{w} was divided into 18 fractions by Middle pressure liquid chromatograph (MPLC), and the 18 fractions obtained by MPLC were purified by HPLC. The qualitative resolution of LCMS and NMR was used at the end. The results are shown in Figure 11. The MPLC system uses Polygoprep C18 tubular column, HPLC system uses Lichrospher Select B and Kromasil C18 tubular columns. The results of qualitative resolution are shown in Figure 11.

**Table 3. Gradient elution process of HPLC separation used for Monstera epipremnoides extract**

| Time (min) | Solvent (A): 5 mM Ammonium formate (AFP) + 0.1% Formic acid | Solvent (B): Methanol: acetonitrile (1:1) + 5 mM AFP + 0.1% Formic acid |
|---|---|---|
| 4 | 73% | 27% |
| 9 | 59% | 41% |
| 10 | 56% | 44% |
| 13 | 48% | 52% |
| 16 | 40% | 60% |
| 20 | 29% | 71% |
| 24 | 18% | 82% |

### EXAMPLE 3

### Monstera epipremnoides extract promotes the regeneration of neurites

The fetal rat of a rat pregnant for 18 days was taken out in this experiment. The fetal rat's brain was divided into cerebral cortex and hippocampus. The cortex and hippocampus were dissociated into cortical neurons and hippocampal neurons, which were incubated in a 48-well plate; this day was called Day *in vitro* 0 (DIV0). Cytosine beta-D-arabinoside (AraC) was added in on DIV2 to inhibit the proliferation of glial cells. The neurons were injured by micropipette tips on DIV8, and treated with 0.1µg/µl plant extracts MeE, MeE_{W} or MeE_{EA}. The regeneration of neurites was observed by fluorescence immunostaining on DIV11, and the results were pictured under Zeiss Observer Z1 microscope.

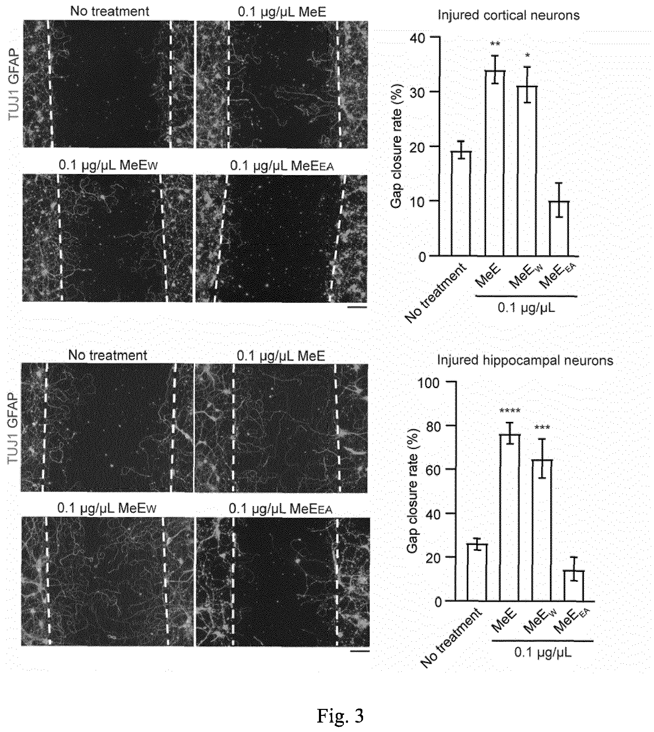

As shown in Figure 3, the white dotted line is the boundary resulted from using micropipette tips to scratch the neurons. According to this experiment, the MeE and MeE_{w} can promote the regeneration of neurites apparently, whereas the MeE_{EA} cannot promote the regeneration of neurites.

Wherein * represents comparison with No treatment. The data were collected from three independent experiments, expressed as data average value ± standard deviation (*: P < 0.05, **: P < 0.01, ***: P < 0.001, ****: P < 0.0001). The scale was 100µm. ZeissObserver Z1 microscope was used for taking pictures in this experiment.

### EXAMPLE 4

### Monstera epipremnoides extract promotes recovery of behavioral function of a rat with cerebral trauma

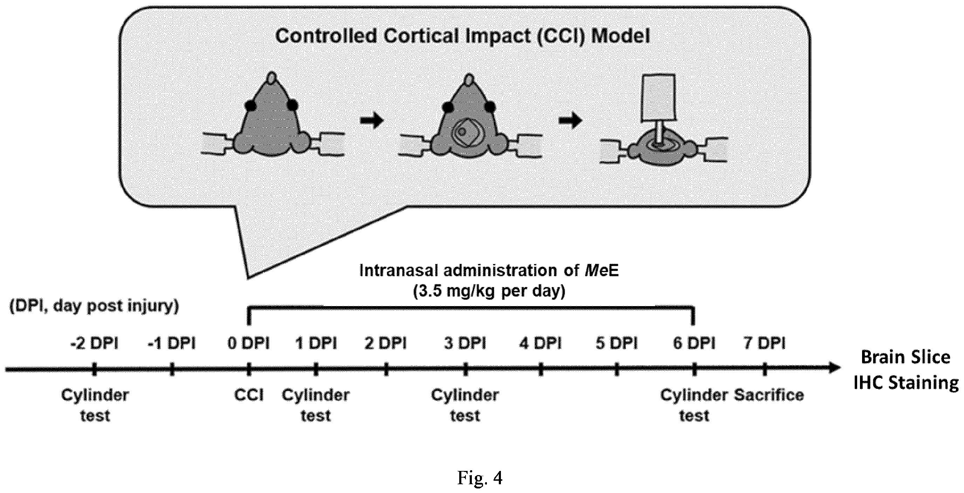

As shown in Figure 4, the effect of MeE on the behavioral function recovery of the living rat with brain trauma was observed. The controlled cortical impact (CCI) model was used in this example. The mouse's meninges were cut open, and the mouse's brain was impacted by an impactor to simulate the state of cerebral trauma; this day was Day post injury 0 (0 DPI). The mouse's motor function recovery was observed by cylinder test, and the mouse was pre-trained on 2 DPI. The cylinder test was performed on 1 DPI, 3 DPI and 6 DPI. The mouse was treated with MeE via intranasal administration every day, and sacrificed on 7 DPI. The brain of mouse was collected for cryosection staining.

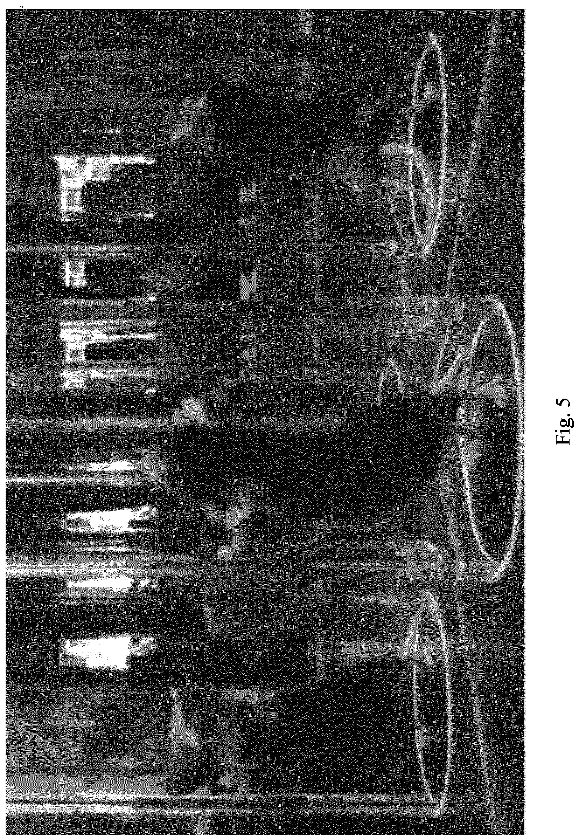

As the region impacted by CCI model was mainly for regulating the motor function, the influence of extract on the mouse behavior repair was observed by cylinder test. As shown in Figure 5, the mouse was placed in a dark cylinder in the cylinder test, and a mirror was placed behind the cylinder. The mouse's behavior could be observed 360°, and the number of rears of the mouse was quantified within 5 minutes. The larger the number of rears, the better the motor function repair.

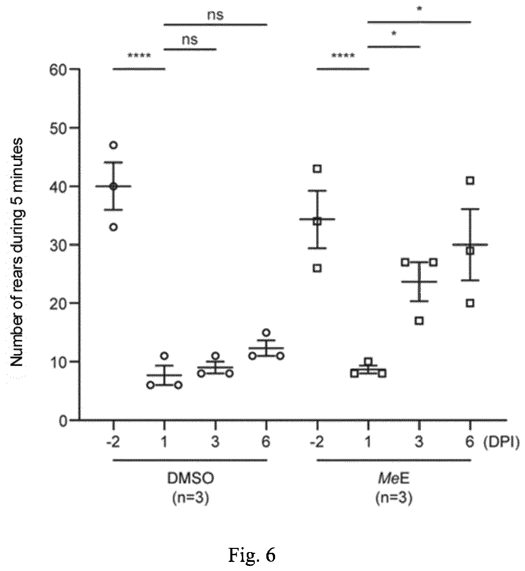

According to the cylinder test result in Figure 6, the comparison result of -2 DPI and 1 DPI shows that the number of rears of the mouse with brain injury decreased significantly, meaning there were motor function deficits. After the extract MeE of the present invention was intranasally administered, the 3 DPI and 6 DPI groups showed apparent motor function recovery.

### EXAMPLE 5

### Monstera epipremnoides extract promotes neuronal regeneration of damaged brain tissue

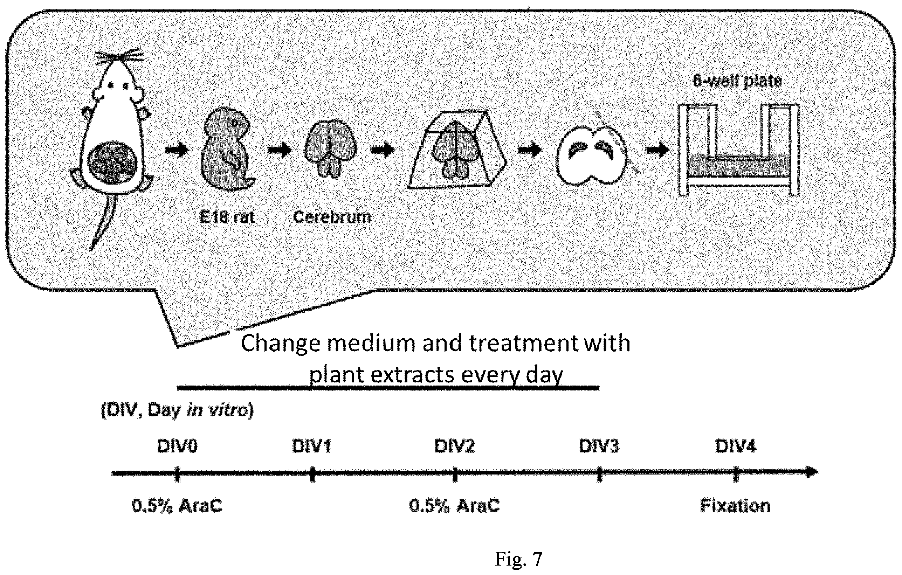

The 3D brain tissue sections were applied to prove the effect of MeE_{W} on promoting the regeneration of brain neurons. The fetal rat's brain was taken out of a rat pregnant for 18 days, and the brain was embedded in low melting temperature agarose. The brain was sliced into 350µm thick section tissues by Leica microtome VT100 for culture. The brain section was injured by a scalpel to observe and quantify neuronal regeneration. 0.5% AraC was added on DIV 0 and DIV 2 to inhibit the proliferation of glial cells, the culture medium was changed and dosed every day. The tissue culture was provided with a solvent every day as control group or provided with MeE_{W}. The neurites and nuclei were labeled by fluorescence immunostaining after 4 days' culture to observe the regeneration of neurites. Figure 7 shows the test flow chart of water extract MeE_{W} for neuronal regeneration in this embodiment.

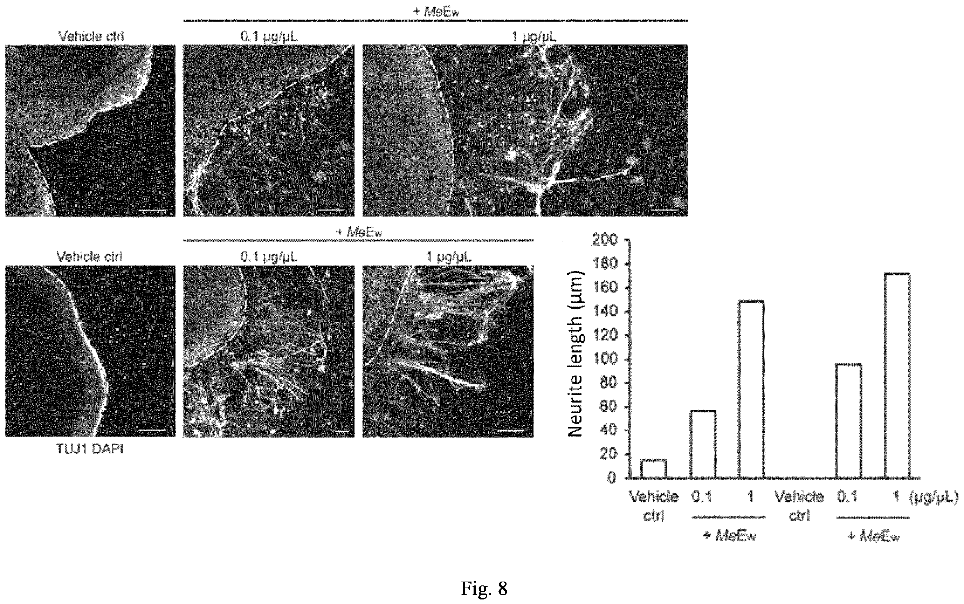

Figure 8 shows the result of injured brain section treated with MeE_{W}. The white dotted line is the gap injured by the scalpel, and the neurite re-growth is on the right side of the dotted line. According to this experiment, MeE_{W} could promote neuronal regeneration. The scale bar was 100µm. Zeiss LSM800 confocal microscope was used for taking images in this experiment. The fluorescence signals were TUJ1 antibody labeled neurons, and GFAP labeled glial cells and DAPI reagent labeled nuclei. The TUJ1 signals on the right side of dotted line were regenerated neurites. The white dotted line length and the TUJ1 signal area on the right side of white line were calculated. The TUJ1 signal area was divided by the white dotted line length to obtain the neurite length, so as to quantify the effect of MeE_{W} on promoting the regeneration of neurites. According to the results, the water- or PBS-soluble MeE_{W} could promote the neurite regeneration of brain tissue.

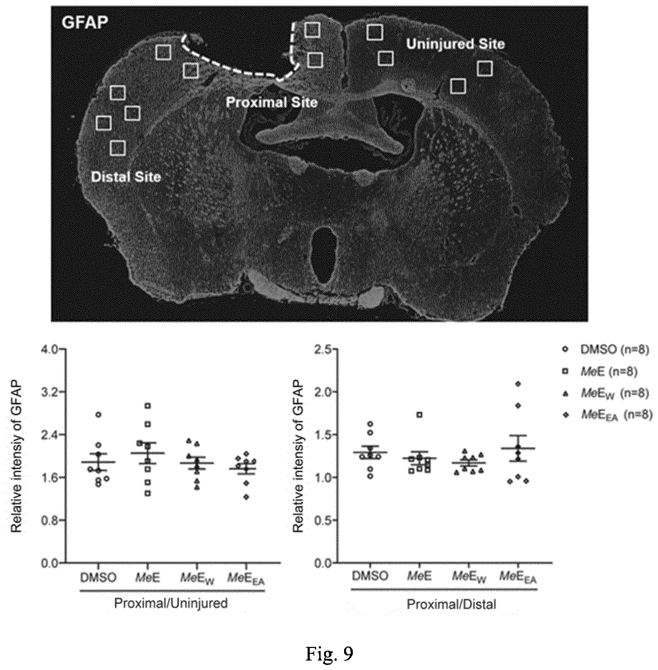

As shown in Figure 9, brain injury leads to inflammatory response, which activates the glial cells to gather around the injured site and forms glial scars that inhibit the regeneration of neurites. Therefore, after the mouse was intranasally administrated with MeE_{W}, it was sacrificed. Its brain was frozen and sectioned. The intensity of GFAP signals, which labelled the glial cells, was calculated in the proximal site, distal site, and the uninjured site respectively, so as to observe whether MeE_{W} had an effect on glial cells. According to the results, the addition of MeE_{w} did not affect glial cells.

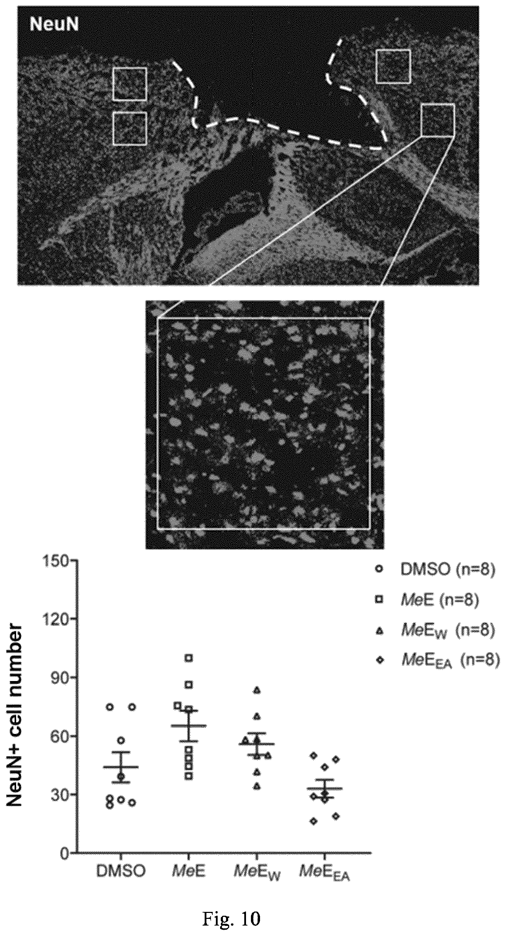

Whether MeE_{w} affects the quantity of neurons around the injured area was observed in Figure 10. The neuron cell nuclei were marked by NeuN, and the number of neurons around the injured area was calculated. The result shows that MeE and MeE_{W} could increase the quantity of neurons around the injured area.

To sum up, the present invention used Monstera epipremnoides of Araceae as a representative for tests, the result proves that the Monstera epipremnoides extract could promote neuronal regeneration after brain trauma; and that the Monstera epipremnoides extract could promote the regeneration of neurite of cortical neurons and hippocampal neurons after brain trauma. Moreover, the results verify that Monstera epipremnoides extract could promote the motor functional recovery of the mouse with brain trauma, and the Monstera epipremnoides extract had no direct effect on glial cells.

All examples provided herein are intended for pedagogical purposes of aiding the reader in understanding the invention and the concepts contributed by the inventors to further the art, and are not to be construed as limitations to such specifically recited examples and conditions, nor does the organization of such examples in the specification relate to a showing of the superiority or inferiority of the invention. Although one or more embodiments of the present invention have been described in detail, it should be understood that the various changes, substitutions, and alterations could be made hereto without departing from the spirit and scope of the invention.

It is intended that the specification and examples be considered as examples only, with a true scope and spirit of the invention being indicated by the following claims.

## Claims

1. Use of Araceae extract in the preparation of drugs for treating nerve injury.

2. The use according to Claim 1, wherein the Araceae extract is extracted by a solvent, wherein the solvent comprises water, ethyl acetate or methanol.

3. The use according to Claim 1, wherein the extract is a crude extract, water extract or an ethyl acetate extract.

4. The use according to Claim 1, wherein the Araceae extract comprises the extract of a Monstera, a Rhaphidophora, a Philodendron, an Epipremnum or a Dieffenbachia.

5. The use according to Claim 4, wherein the Monstera extract is a Monstera epipremnoides extract.

6. The use according to Claim 1, wherein the nerve injury is central nervous system and peripheral nervous system injuries.

7. The use according to Claim 1, wherein the nerve injury refers to a neuronal injury.

8. The use according to Claim 7, wherein the neuronal injury comprises cortical neurons or hippocampal neurons injury.

9. The use according to Claim 1, wherein the said treatment refers to neuronal regeneration, increase in number of neurons or nerve repair.

10. The use according to Claim 1, wherein the Araceae extract can penetrate the blood-brain barrier and enter the brain.

11. An Araceae extract, comprising a compound A of chromatogram with at least one peak at 13.70 to about 14.10 minutes retention time determined by HPLC.

12. The Araceae extract according to Claim 11, the Araceae extract further comprises a compound B of chromatogram with at least one peak at 4.4 to about 4.6 minutes retention time.

13. The Araceae extract according to Claim 11, the Araceae extract further comprises a compound C of chromatogram with at least one peak at 4.8 to about 5 minutes retention time.

14. The Araceae extract according to Claim 11, the Araceae extract further comprises a compound D of chromatogram with at least one peak at 10 to about 11 minutes retention time.

15. The Araceae extract according to Claim 11, the Araceae extract is prepared through the following steps:
(A) the Araceae is extracted with an organic solvent, and filtered to obtain a filtrate A, after that the filtrate A is concentrated by rotary evaporation to obtain an extract A; and
(B) the extract A is analyzed at 210-400 nm by HPLC, the HPLC is performed in the following conditions, the mobile phases include a mobile phase A and a mobile phase B, the gradient of 4 min is mobile phase A volume fraction 73% and mobile phase B volume fraction 27%; the gradient of 9 min is mobile phase A volume fraction 59% and mobile phase B volume fraction 41%; the gradient of 10 min is mobile phase A volume fraction 56% and mobile phase B volume fraction 44%; the gradient of 13 min is mobile phase A volume fraction 48% and mobile phase B volume fraction 52%; the gradient of 16 min is mobile phase A volume fraction 40% and mobile phase B volume fraction 60%; the gradient of 20 min is mobile phase A volume fraction 29% and mobile phase B volume fraction 71%; the gradient of 24 min is mobile phase A volume fraction 18% and mobile phase B volume fraction 82%; wherein the mobile phase A is 5 mM Ammonium formate (AFP) and 0.1% Formic acid; wherein the mobile phase B is 1:1 of methanol : acetonitrile and 5 mM AFP combination with 0.1% Formic acid.

16. The Araceae extract according to Claim 15, wherein the organic solvent of step (A) is ethyl acetate or methanol.

17. The Araceae extract according to Claim 15, wherein the extract is a water extract or an ethyl acetate extract.

18. The Araceae extract according to Claim 11, wherein the Araceae extract comprises the extract of a Monstera, a Rhaphidophora, a Philodendron, an Epipremnum or a Dieffenbachia.

19. The Araceae extract according to Claim Claim 18, wherein the Monstera extract is a Monstera epipremnoides extract.
